(19) Europäisches Patentamt
European Patent Office
Office européen des brevets

(11) Veröffentlichungsnummer: **0 028 338**
**B2**

(12) **NEUE EUROPÄISCHE PATENTSCHRIFT**

(45) Veröffentlichungstag der neuen Patentschrift: **26.02.86**

(21) Anmeldenummer: **80106302.5**

(22) Anmeldetag: **16.10.80**

(51) Int. Cl.[4]: **C 07 C 118/00,** C 07 C 119/048, C 07 C 125/065, C 07 C 125/073, C 07 C 125/077

(54) **Verfahren zur Herstellung von aromatischen Di- und/oder Polyisocyanaten.**

(30) Priorität: **20.10.79 DE 2942503**

(43) Veröffentlichungstag der Anmeldung:
**13.05.81 Patentblatt 81/19**

(45) Bekanntmachung des Hinweises auf die Patenterteilung:
**18.01.84 Patentblatt 84/3**

(45) Bekanntmachung des Hinweises auf die Entscheidung über den Einspruch:
**26.02.86 Patentblatt 86/9**

(84) Benannte Vertragsstaaten:
**BE DE FR GB IT NL**

(56) Entgegenhaltungen:
**EP-A-0 018 581**
**EP-A-0 018 583**
**US-A-2 806 051**
**US-A-3 919 279**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Towae, Friedrich, Dr., Parkstrasse 22, D-6700 Ludwigshafen (DE)**

EP 0 028 338 B2

**Beschreibung**

Die Erfindung betrifft ein Verfahren zur Herstellung von aromatischen Di- und/oder Polyisocyanaten durch Umsetzung von

A. primären aromatischen Di- und/oder Polyaminen mit O-Alkylcarbamidsäureestern in Abwesenheit oder vorzugsweise in Gegenwart von Katalysatoren, wobei als Katalysator mindestens eine Verbindung verwendet wird, die ein oder mehrere Kationen von Metallen aus den Gruppen IA, IB, IIA, IIB, IIIA, IIIB, IVA, IVB, VA, VB, VIB, VIIB und VIIIB des Periodensystems besitzt, und gegebenenfalls Harnstoff und Alkohol bei erhöhten Temperaturen zu Aryl-di- und/oder -polyurethanen und

B. Spaltung der erhaltenen Aryl-di- und/oder -polyurethane in Gegenwart eines Katalysators bei Temperaturen von 175° bis 600°C in aromatische Di- und/oder Polyisocyanate.

Zur Herstellung von aromatischen Di- und/oder Polyisocyanaten werden üblicherweise primäre aromatische Di- und/oder Polyamine mit Phosgen in die entsprechenden Carbamidsäurechloride übergeführt und diese danach thermisch, gegebenenfalls in Gegenwart von Katalysatoren, unter Freisetzung von Chlorwasserstoff gespalten.

Problematisch bei dieser Verfahrensweise sind besonders der hohe Umsatz von Chlor über Phosgen und Carbamidchlorid in Chlorwasserstoff, die Toxizität des Phosgens und die Korrosivität der Reaktionsgemische sowie die Labilität der in der Regel eingesetzten Lösungsmittel, verbunden mit kostspieligem Material- und sicherheitstechnischem Aufwand.

Es hat daher nicht an Versuchen gefehlt, aromatische Di- und/oder Polyisocyanate ohne Chlor und Phosgen herzustellen.

Nach Angaben der GB-PS 1 025 436 können organische Diisocyanate direkt durch Umsetzung von Kohlenmonoxid mit organischen Nitroverbindungen in Gegenwart von Edelmetallen als Katalysatoren in Abwesenheit von Feuchtigkeit und Wasserstoff bei erhöhten Temperaturen und erhöhtem Druck erhalten werden. Geeignete Katalysatoren sind ferner nach DE-OS 1 815 517 (US 3 576 835) Komplexe oder Mischungen aus einer heteroaromatischen Verbindung und mindestens einem Edelmetallhalogenid sowie nach De-OS 19 31 212 (US 3 654 279 und US 3 781 321) Verbindungen der allgemeinen Formel $PdI(CO)X_2$, in der L eine Lewissäure und X ein Halogenatom bedeuten.

Da die Katalysatorkosten unwirtschaftlich hoch, die Reaktionsbedingungen technisch kritisch und die Isocyanatausbeuten unbefriedigend sind, haben die genannten Verfahren bisher keine technische Bedeutung erlangt. Sie kommen insbesondere für Polyisocyanate, wie Mischungen aus Diphenylmethandiisocyanaten und Polyphenyl-polymethylen-polyisocyanaten (roh-MDI) nicht in Betracht, da es geeignete Synthesen zur Herstellung der gewünschten poly-nitroaromaten nicht gibt.

Zur Beseitigung dieser Nachteile wurde vorgeschlagen, die aromatischen Amine bzw. Nitroverbindung zunächst in Urethan überzuführen und diese thermisch zu spalten.

Die DE-OS 21 60 111 (US 3 763 217) und DE-OS 27 16 540 (US 4 100 351) beschreiben Verfahren zur Herstellung von Arylurethanen aus Arylaminen, wie Toluylendiamin, bzw. N-Arylamiden und Dimethylcarbonat in Gegenwart einer Lewissäure. Dimethylcarbonat muß jedoch aus Phosgen und Methanol oder nach neueren Vorschlägen aus Kohlenmonoxid und Methanol durch technisch schwierig durchführbare Cooxidation produziert werden und ist im Vergleich zu Phosgen noch sehr teuer. Nachteilig sind ferner die recht geringe Reaktionsgeschwindigkeit und die konkurrierende Bildung von N-Alkylarylaminen.

Nach Angaben der DE-OS 15 68 044 (US-PS 3 467 694) können Urethane durch Umsetzung von organischen Nitroverbindungen, Kohlenmonoxid und hydroxylhaltigen Verbindungen in Gegenwart eines Katalysators, der aus einem Edelmetall und einer Lewissäure besteht, unter praktisch wasserfreien Bedingungen in Abwesenheit von Wasserstoff bei erhöhtem Druck und Temperaturen über 150°C hergestellt werden. Gemäß der DE-OS 23 43 826 (US 3 895 054) werden Urethane aus Hydroxylgruppen haltigen Verbindungen, Kohlenmonoxid und Nitro-, Nitroso-, Azo- und Azoxy-Gruppen aufweisenden Verbindungen in Gegenwart von Schwefel, Selen, einer Schwefel- und/oder Selenverbindung und mindestens einer Base und/oder Wasser hergestellt. Die DE-OS 26 23 694 (US 4 080 365) beschreibt die Herstellung aromatischer Urethane aus den obengenannten Ausgangsverbindungen in Gegenwart von Selen enthaltenden Katalysatorsystemen sowie speziellen aromatischen Amino- und Harnstoffverbindungen.

Aber auch diese Verfahren weisen noch schwerwiegende Nachteile auf. Das toxische Kohlenmonoxid als Ausgangsstoff und Katalysatoren, die toxisch sind oder im Laufe der Umsetzung toxische Verbindungen bilden, beispielsweise Selen- und Schwefelwasserstoff, oder Katalysatoren, welche sehr teuer und schwierig rückführbar sind, wie Palladium, erfordern einen hohen technischen Aufwand mit kostspieligen Sicherheitsmaßnahmen. Sie sind darüberhinaus ebensowenig universell anwendbar wie die Direktsynthese von Isocyanaten aus Nitroaromaten, insbesondere nicht für Polyurethane vom Typ der (Poly)-methylen-polyphenylurethane, den potentiellen Vorprodukten für eines der technisch bedeutendsten Polyisocyanate, roh-MDI. Das aus Nitrobenzol, Kohlenmonoxid und Alkohol, gemessen am technischen Stand, vorteilhaft zugängliche Phenylurethan läßt sich zwar nach DE-PS 10 42 891 (US 29 46 768) und DE-OS 28 32 379 (US 41 46 727) durch Kondensation mit Formaldehyd in Gegenwart großer Mengen einer starken Säure zweistufig zu Methylen-bis-phenylurethan und Polymethylen-polyphenylurethan (roh-MDu) verarbeiten, dessen Thermolyse ein roh-MDI liefert. Das

Verfahren ist jedoch technisch nicht befriedigend flexibel und kompliziert. Begrenzt ist besonders die Flexibilität bezüglich der gezielten Einstellung von Isomerenverhältnissen in den Produkten; man erhält im Spaltprodukt einen für bedeutende Anwendungsbereiche nachteilig hohen Anteil an 2,4'- und 2,2'-Methylen-bis-phenylisocyanaten.

Wie bereits dargelegt wurde, sind N-substituierte Urethane thermisch in der Gasphase oder in flüssiger Phase in Isocyanate spaltbar. Bei der thermischen Spaltung treten gleichzeitig verschiedene unerwünschte Nebenreaktionen auf. Gennannt seien beispielsweise die Decarboxylierungsreaktion der Urethane, die von der Bildung primärer und sekundärer Amine sowie Olefine begleitet sein kann; Umsetzungen zwischen dem gebildeten Isocyanat und Urethan zu Allophanaten bzw. Amin zu Harnstoffen und Polymerisation der Isocyanate zu Isocyanuraten.

Die Pyrolyse der Urethane in der Dampfphase — die Dampfphase ist hierbei so definiert, daß das Produktgemisch gegebenenfalls einschließlich des Lösungsmittels, nach der Spaltung in der Dampfphase vorliegt, unabhängig davon, ob die zu spaltenden Urethane gasförmig, flüssig oder fest zugeführt werden — wird nach Angaben der DE-OS 19 44 719 (GB-PS 1 247 451) bei Temperaturen von 400° bis 600°C in Gegenwart einer Lewissäure als Katalysator durchgeführt, wobei das Isocyanat und der Alkohol durch fraktionierte Kondensation getrennt werden. Toluylen-diisocyanat wird z.B. durch Pyrolyse von Toluylen-2,4-diäthylurethan in Gegenwart von Eisen-(III)-chlorid erhalten. Nachteile der Reaktion sind u.a. neidrige Ausbeuten, verbunden mit beträchtlichen Mengen eines polymeren Nebenprudukts, Zersetzung des Katalysators und Korrosion der Reaktionsapparatur. In der DE-OS 24 10 505 (US 3 870 739) wird ein Verfahren beschrieben, bei dem das Urethan bei einer Temperatur von 350 bis 550°C und einem Druck von weniger als dem (m+1)fachen des Isocyanatdampfdruckes in einer katalysatorfreien Pyroslysezone innerhalb von 15 Sekunden gespalten wird. Nachteilig an diesem Verfahren ist u.a. daß eine große für die endotherme Spaltung erforderliche Wärmemenge innerhalb einer sehr kurzen Zeit dem pulverförmigen Urethan zugeführt werden muß und ein als Nebenprodukt anfallendes festes Polymer und dessen Abtrennung die Durchführung eines kontinuierlichen Verfahrens erschwert.

Die thermische Spaltung von Urethanen in flüssiger Phase wird beispielsweise in den DE-AS 24 21 503 (US 3 962 302) und DE-AS 25 30 001 (US 3 919 280) beschrieben. Nach Angaben der DE-AS 24 21 503 werden die Urethane in einem inerten Lösungsmittel, wie Alkylbenzolen, linearen und cyclischen Kohlenwasserstoffen und/oder Phthalsäureestern, gelöst und unter Normal- oder Überdruck bei 175° bis 350°C gespalten. Die Isolierung und Trennung des erhaltenen Isocyanats und Alkohols erfolgt mit Hilfe des Lösungsmittels als Trägermittel und/oder unter Verwendung eines Inertgases als Trägermittel. Gemäß DE-AS 25 30 001 werden als Reaktionsmedium höhermolekulare, gegebenenfalls substituierte aliphatische, cycloaliphatische oder aromatische Kohlenwasserstoffe, Äther, Ester oder Ketone verwendet. Zur Trennung der Spaltprodukte wird nur die Destillation genannt, wobei über Kopf Isocyanat, Alkohol und Trägermaterial abdestilliert werden, während das Reaktionsmedium als Boden-fraktion zurückbleibt.

Zur Herstellung von aromatischen Isocyanaten werden die Urethane nach DE-OS 26 35 490 (US 4 081 472) bei Temperaturen von 150°C bis 350°C unter vermindertem Druck mit einer Lösung aus wenigstens einem Metallion, wie Ionen aus Kupfer, Zink, Aluminium, Zinn, Titan, Vanadium, Eisen, Kobalt und Nickel als Katalysator, der in einem Lösungsmittel mit einem Siedepunkt von 200°C in einer Metallkonzentration von wenigstens 0,001 Gew.%, bezogen auf das Lösungsmittel, gelöst ist, in Kontakt gebracht. Die Trennung der erhaltenen Spaltprodukte erfolgt durch fraktionierte Kondensation. Nach den genannten Methoden können Urethane in Abhängigkeit von ihrer Struktur in teilweise sehr guten Ausbeuten in Isocyanate übergeführt werden. Nicht beispielhaft beschrieben in diesen Patentschriften wird die Herstellung von roh-MDI. Im Unterschied zu den beispielhaft aufgeführten Isocyanaten ist Roh-MDI nicht mit Hilfe von Lösungsmitteln als Träger vollständig destillierbar und kann dahr nicht wie beschrieben von Katalysatoren, Lösongsmittel und gegebenenfalls nicht umgesetzten Ausgangsstoffen sowie Verunreinigungen isoliert werden.

Aufgabe der vorliegenden Erfindung war es, aromatische Di- und/oder Polyisocyanate wirtschaftlich und ökologisch vorteilhaft aus leicht zugänglichen Ausgangsstoffen ohne die toxischen Komponenten Phosgen, Chlor und Chlorwasserstoff und ohne kostspielige bzw. toxische Katalysatoren, wie Palladium und Selen, zugänglich zu machen.

Diese Aufgabe wurde gelöst durch ein Verfahren zur Herstellung von aromatischen Di- und/oder Polyisocyanaten in zwei Reaktionsstufen, das dadurch gekennzeichnet ist, daß man

A. primäre aroamtische Di- und/oder Polyamine mit O-Alkylcarbamidsäureestern in Gegenwart oder Abwesenheit von Katalysatoren und gegebenenfalls Harnstoff und Alkohol bei erhöhten Temperaturen zu Aryl-di- und/oder -polyurethanen umsetzt und den dabei gebildeten Ammoniak gegebenenfalls abtrennt und

B. die erhaltenen Aryl-di- und/oder -polyurethane durch thermische Spaltung in Gegenwart von Katalysatoren bei Temperaturen von 175° bis 600°C in aromatische di- und/oder Polyisocyanate überführt.

Das erfindungsgemäße Verfahren kann schematisch allgemein durch die Gleichungen (I) und (III) oder (II) und (III) veranschaulicht werden:

$$Ar(-NH_2)_n + nH_2N-COOR \rightarrow Ar(NHCOOR)_n + nNH_3 \quad (I)$$

Nach einer bevorzugten Ausführungsform wird die Umsetzung in Gegenwart von Harnstoff und Alkohol nach Gleichung (II) durchgeführt

$$Ar(-NH_2)_n + a\ H_2N-CO-NH_2 + b\ ROOC-NH_2 + a\ ROH \downarrow Ar(-NHCOOR)_n + (2a + b)NH_3 \qquad (II)$$

Die thermische Spaltung der Urethane erfolgt nach Gleichung (III)

$$Ar(-NHCOOR)_n\ \Delta \rightarrow AR(NCO)_n + ROH \qquad (III$$

Speziell für Diphenylmethan-diisocyanate und roh-MDI ergeben sich die Formulierungen (IV) und (V)

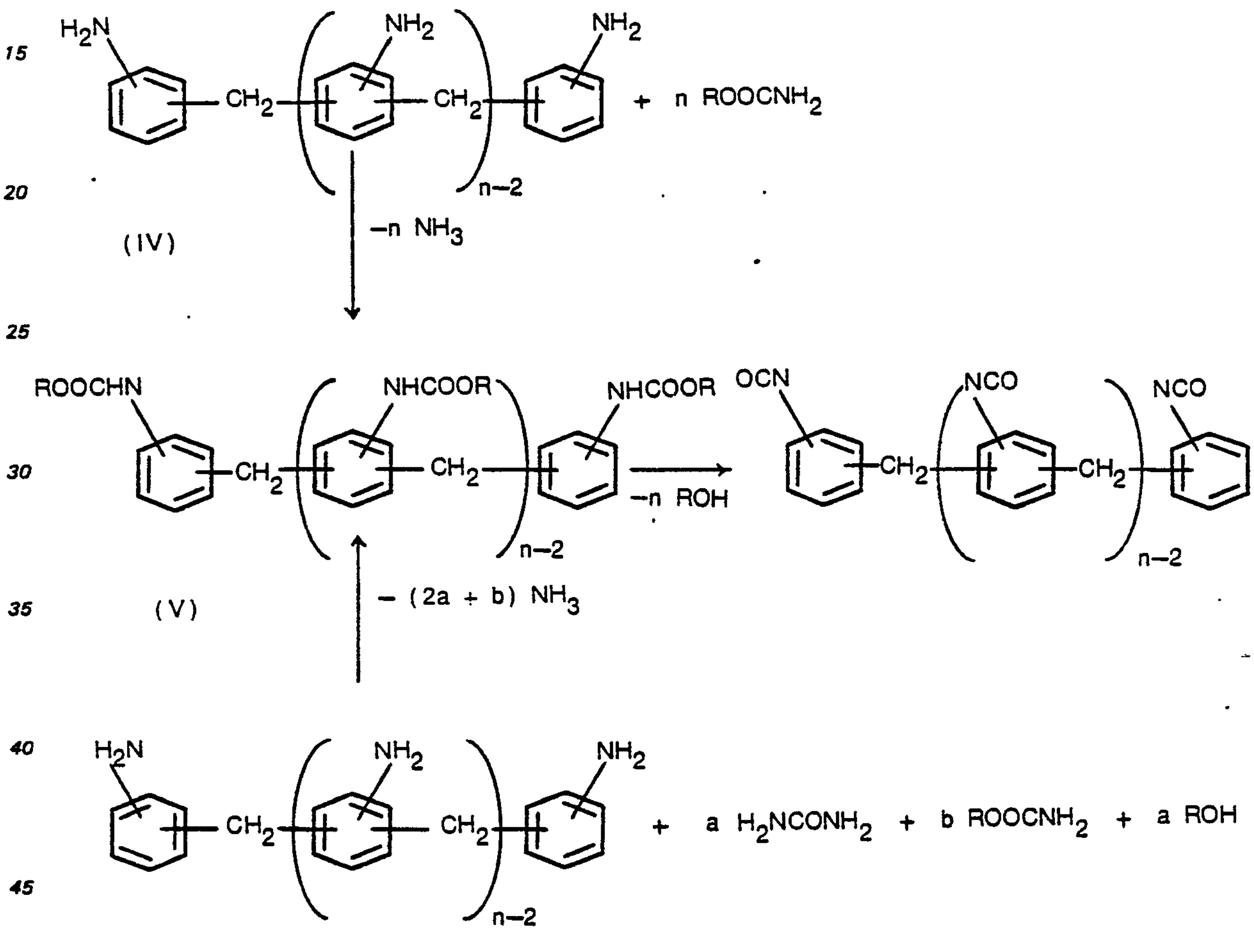


(IV)

(V)

In den Gleichungen bedeuten n, a und b ganze Zahlen, wobei n für 2 bis 8 oder höher, vorzugsweise 2 bis 6 steht und gemäß Gleichung (II) und (V) a+b=n und a:n=1,5 bis 0 sind.

Die Bildung von Aryl-di und/oder -polyurethanen, insbesondere in einem Verfahrensschritt und in guten Ausbeuten, ist überraschend.

Aus Diaminen und Harnstoff entstehen bekanntlich bei Temperaturen über 100°C hochmolekulare, verspinnbare Kondensationsprodukte (DE-PS 896 412). Hochmolekulare Polyharnstoffe, beispielsweise mit Molekulargewichten von 8 000 bis 10 000 und größer werden auch erhalten, wenn man Diurethane mit Diaminen bei Temperaturen von umgefähr 150°C bis 300°C kondensiert (US-PS 2 181 663 und US-PS 2 568 885). Da Mono- und Polyurethane außerdem thermisch in Isocyanate, Alkohole und gegebenenfalls Olefine, Kohlendioxid, Harnstoff und Carbodiimide gespalten werden, wobei die erhaltenen Spaltprodukte wieder zahlreiche Folgeprodukte, z.B. Biurete, Allophanate, Isocyanurate, Polycarbodiimide, u.a. bilden können, (J.A.C.S. *80,* 5495 (1958) und J.A.C.S. *78,* 1946 (1956)) konnte nicht erwartet werden, daß unter zum Teil ähnlichen Reaktionsbedingungen nach dem erfindungsgemäßen Verfahren Aryl-di- und/oder -polyurethane in sehr guten Ausbeuten erhalten werden.

Es ist insbesondere überraschend, daß Aryl- di- und/oder -polyurethane in guten Ausbeuten aus aromatischen primären Aminen und O-Alkylcarbamidsäureestern erfindungsgemäß katalytisch bereits ohne Zusatz von Alkohol erhalten werden, wobei überschüssige O-Alkylcarbamidsäureester nicht zu wesentlichen Zersetzungen und Polykondensationen führen und die Reaktion durch Zusatz der genannten

Katalysatoren stark beschleunigt und in ihrer Selektivät noch verbessert werden kann. Überraschend ist ferner, daß die Umsetzung mit Harnstoff und Alkohol in Gegenwart mindestens eines der genannten Katalysatoren bereits bei tieferen Temperaturen technisch erheblich effektiver wird, wenn sie zusammen mit größeren Mengen O-Alkylcarbamidsäureester durchgeführt wird. Es ist nicht erforderlich O-Alkylcarbamidsäureester separat herzustellen. In einer leicht praktikablen, vorzugsweise zur Anwendung kommenden Ausführungsform wird vorgefertigter O-Alkylcarbamidsäureester zusammen mit Harnstoff und Alkohol eingesetzt und nach weitgehender bis vollständiger Umsetzung des primären aromatischen Di- und/oder Polyamins durch Destillation abgetrennt und gegebenenfalls zurückgeführt. Die Herstellung der Aryl-di- und/oder polyurethane nach dem erfindungsgemäßen Reaktionsschritt kann auch kontinuierlich durchgeführt werden.

Für die Umsetzung mit O-Alkylcarbamidsäureestern in Abwesenheit oder vorzugsweise in Gegenwart von Katalysatoren und gedgebenenfalls Harnstoff und Alkohol eignen sich gegebenenfalls substituierte primäre aromatische Di- und/oder Polyamine. Im einzelnen seien beispielshaft gennant:

Aromatische Diamine, wie 1,3- und 1,4-Diaminobenzol; durch eine Nitro-, Methyl-, Äthyl-, n-Propyl-, Isopropyl-, n-Butyl, Isobutyl-, sek-Butyl-, tert.-Butyl-, Methoxy-, Äthoxy-, n-Propoxy-, Isopropoxy-, n-Butoxy-, Isobutoxy-, sek.-Butoxy-, tert-Butoxygruppe oder ein Halogenatom, vorzugsweise ein Fluor- und/oder Chloratom, in 2- und/oder 4-Stellung substituiertes 1,3-Diaminobenzol oder in 2-Stellung substituiertes 1,4-Diaminobenzol, 1,5- und 1,8-Diaminonaphthalin, 4,4'-Diaminodiphenyl, 2,2'-, 2,4'- und 4,4'-Diamino-diphenylmethan und die entsprechenden Isomerengemische und aromatische Polyamine, wie 1,3,5-Triamino-benzol, 2,4,6-Triaminotoluol, 1,3,5-Triaminonaphthalin und Polyphenyl-polymethylenpolyamine sowie Mischungen aus Diamino-diphenylmethanen und Polyphenyl-polymethylenpolyaminen, die nach bekannten Verfahren durch Kondensation von Anilin und Formaldehyd in Gegenwart von vorzugsweise Mineralsäuren als Katalysatoren erhalten werden.

Besonders bewährt haben sich und daher vorzugsweise verwendet werden 2,4- und 2,6-Diaminotoluol und die entsprechenen Isomerengemische, 1,5-Diaminonaphthalin, 3,3'-Ditoluylen-4,4-diamin, 2,2'- 2,4'- und 4,4'-Diaminodiphenylmethan und die entsprechenden Isomerengemische und Mischungen aus Diaminodiphenylmethan-Isomeren und Polyphenyl-polymethylen-polyaminen.

Bei der Reaktion werden die Aminogruppen in Alkoxycarbonylaminogruppen übergeführt, unabhängig davon, ob die übrigen Substituenten unverändert erhalten bleiben oder ebenfalls umgewandelt werden.

Geeignete O-Alkylcarbamidsäureester besitzen die Formel $H_2N—COOR$, in der R einen gegebenenfalls substituierten aliphatischen, cycloaliphatischen oder aromatisch-aliphatischen Rest bedeutet. In Betracht kommen beispielsweise O-Alkylcarbamidsäureester auf Basis von primären aliphatischen Monoalkoholen mit 1 bis 20 Kohlenstoffatomen, vorzugsweise 1 bis 10 Kohlenstoffatomen, wie Carbamidsäure-methylester, -äthylester, -propylester, -n-butylester, -isobutylester, -2- und -3-methylbutylester, -neopentylester, -pentylester, -2-methyl-pentylester, -n-hexylester, -2-äthylhexylester, -heptylester, -n-octylester, -n-nonylester, -n-decylester und -n-dodecylester, 2-phenylpropylester und -benzylester, auf Basis von sekundären aliphatischen und cycloaliphatischen Monoalkoholen mit 3 bis 15 Kohlenstoffatomen, vorzugsweise 3 bis 6 Kohlenstoffatomen, wie Carbamidsäure-isopropylester, -sek.-butylester, -sek.-iso-amylester, -cyclopentylester, -cyclohexylester, tert.-butyl-cyclohexylester und -bicyclo(2.2.1)-heptylester. Vorzugsweise verwendet werden Carbamidsäure-methylester, -äthylester, -propylester, -butylester, -isobutylester, -2- und -3-methylbutylester, -pentylester, -hexylester, -2-äthylhexylester, -heptylester, -octylester und -cyclohexylester.

Zur Herstellung der Aryl-di- und/oder -polyurethane werden die obengenannten primären aromatischen Di- und/oder Polyamine mit den O-Alkylcarbamidsäureester und gegebenenfalls Alkoholen in solchen Mengen umgesetzt, daß das Verhältnis von $NH_2$-Gruppen der primären aromatischen Amine zu O-Alkylcarbamidsäureestern zu Alkohol 1:0,5 bis 20:0 bis 100 vorzugsweise 1:1 bis 10:1 bis 30 und insbesondere 1:1 bis 6:2 bis 20 beträgt.

Wie bereits ausgeführt wurde, werden zur Herstellung der Aryl-di- und/oder -polyurethane in einer der bevorzugten Varianten neben O-Alkylcarbamidsäureester zusätzlich Harnstoff und Alkohol mitverwendet, wobei das Verhältnis von $NH_2$-Gruppen der primären aromatischen Amine zur Summe aus O-Alkylcarbamidsäureester und Harnstoff ebenfalls 1:0,5 bis 20, vorzugsweise 1:1 und insbesondere 1:1 bis 6 beträgt, das Molverhältnis von Harnstoff zu Aminogruppen der primären aromatischen Amine gleich oder kleiner als 1,5, vorzugsweise 1,25 bis 0,75 und das Molverhältnis Harnstoff zu Alkohol gleich oder kleiner als 1 sind.

Harnstoff wird zweckmäßigerweise in handelsüblicher Form und Reinheit eingesetzt.

Als Alkohole können beliebige, gegebenenfalls substituierte, primäre oder sekundäre aliphatische Alkohole sowie Mischungen davon verwendet werden. Vorzugsweise wird der den O-Alkylcarbamidsäureestern entsprechende Alkohol eingesetzt.

In Betracht kommen beispielsweise primäre aliphatische Alkohole mit 1 bis 20 Kohlenstoffatomen, vortzugsweise 1 bis 10 Kohlenstoffatomen, wie Methanol, Äthanol, Propanol, n-Butanol, 2-Methylbutanol, n-Pentanol, Neopentylalkohol, 2-Methyl-pentanol, n-Hexanol, n-Heptanol, n-Octanol, Nonanol, n-Decanol und n-Dodecanol, sekundäre aliphatische und cycloaliphatische Alkohole mit 3 bis 15 Kohlenstoffatomen, vorzugsweise 3 bis 6 Kohlenstoffatomen, wie Isopropanol, sec.-Butanol, sec.-Isoamylalkohol, Cyclopentanol, 2,3- oder 4-Methyl-cyclohexanol, Cyclohexanol und Bicyclo-(2,2,1)-heptanol. Vorzugsweise

verwendet werden als Monoalkohole Methanol, Äthanol, Propanol, n-Butanol, Isobutanol, 2-Äthylbutanol, 2- und 3-Methylbutanol, n-Pentanol, n-Hexanol, 2-Äthyl-hexanol, Heptanol, Octanol und Cyclohexanol.

Die Alkohole können gegebenenfalls mit anderen unter den Reaktionsbedingungen inerten organischen Lösongsmitteln gemischt werden.

Zur Erhöhung der Reaktionsgeschwindigkeit und Verbesserung der Ausbeute werden die Aryl-di- und/oder -polyurethane nach dem erfindungsgemäßen Verfahren vorzugsweise in Gegenwart mindestens eines Katalysators hergestellt. Als Katalysatoren eignen sich anorganische oder organische Verbindungen, die ein oder mehrere, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14th. Edition, publiziert von Chemical Rubber Publishing Co. 2310 Superior Ave. N.E. Cleveland, Ohio, enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate. Beispielhaf gennant seien die Kationen folgender Metalle: Lithium, Natrium, Kalium, Magnesium, Calcium, Aluminium, Gallium, Zinn, Blei, Wismut, Antimon, Kupfer, Silber, Gold, Zink, Quecksilber, Cer, Titan, Vanadium, Chrom, Molybdän, Mangan, Eisen, Kobalt und Nickel. Vorzugsweise Werwendung fiden die Kationen von Lithium, Calcium, Aluminium, Zinn, Wismut, Antimon, Kupfer, Zink, Titan, Vanadium, Chrom, Molybdän, Mangan, Eisen und Kobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen gennant: Lithiummethanolat, Lithiummäthanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminium-isobutylat, Aluminiumtrichlorid, Wismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titantetrabutanolat, Titantetrachlorid, Titantetraphenolat, Titannaphthenat, Vanadium-(III)-chlorid, Vanadiumacetonylacetat, Chrom-(III)-chlorid, Molybdän-(IV)-oxid, Molybdän-acetylacetonat, Wolfram-(VI)-oxid, Mangan-(II)-chlorid, Mangan-(II)-acetat, Mangan-(II)-acetat, Eisen-(II)-acetat, Eisen-(III)-acetat, Eisenphosphat, Eisenoxalat, Eisen-(III)-chlorid, Eisen-(III)-bromid, Kobaltacetat, Kobaltchlorid, Kobaltsulfat, Kobaltnaphthenat, Nickelchlorid, Nickelacetat und Nickelnaphthenat sowie deren Gemische.

Die Katalysatoren werden zweckmäßigerweise in Mengen, die 0,000 1 bis 0,1, vorzugsweise 0,000 5 bis 0,05 Äquivalenten des metallkations, bezogen auf die $NH_2$-Gruppen der aromatischen Di- und/oder Polyamine entsprechen, verwendet. Die Metallionen können auch gebunden an Ioneaustauscher in heretogener Phase eingesetzt werden.

Die Umsetzung wird bei erhöhten Temperaturen, beispielsweise bei Temperaturen von 100° bis 25°C, vorzugsweise 130° bis 210°C und insbesondere 150° bis 190°C und Drücken von 0,1 bis 120 bar, vorzugsweise 0,5 bis 60 bar, insbesondere 1 bis 40 bar, durchgeführt, wobei es sich als vorteilhaft erwiesen hat, das entstehende Ammoniak aus der Reaktionsmischung beispielsweise durch Destillation, abzutrennen. Bei gegebener Temperatur wird die Umsetzung dann vorzugsweise unter einem Druck durchgeführt, bei dem der entstehende Ammoniak selektiv aus dem Reaktionsgemisch abdestilliert werden kann. Die entsprechenden Werte könnon Tabellen mit physikalischen Kenndaten des Ammoniaks und der Alkohole entnommen werden. Für den genannten Temperaturbereich ergeben sich Reaktionszeiten von 0,5 bis 100 Stunden, vorzugsweise von 1 bis 50 Stunden und insbesondere 2 bis 25 Stunden.

Die Aryl-di- und/oder -polyurethane werden nach dem erfindungsgemäßen Verfahren zweckmäßigerweise wie folgt hergestellt: Die primären aromatischen Di- und/oder Polyamine, O-Alkylcarbamidsaüreester und gegebenenfalls Alkohole bzw. Di- und/oder Polyamine, O-Alkylcarbamidsaüreester, Harnstoff und Alkohole werden in den genannten Mengenverhältnissen gemischt und gegebenenfalls in Gegenwart von mindestens einem Katalysator in einem Reaktionsgefäß, ausgestattet mit einer Vorrichtung zur Abtrennung des Ammoniaks, gegebenenfalls unter Rühren erhitzt. Das entstehende Ammoniak kann nach beendeter Umsetzung abgetrennt werden; vorzugsweise wird es jedoch bereits im Laufe der Umsetzung kontinuierlich oder absatzweise abdestilliert. Hierbei kann es zweckmäßig sein, insbesondere bei der Umsetzung in Gegenwart von niedermolekularen Alkoholen unter Druck, das Ammoniak mit Hilfe eines unter den Reaktionsbedingungen inerten Schleppmittels, beispielsweise eines Gases, wie Stickstoff, oder zusammen mit einem Teil des Alkohols abzutrennen. Aus der erhaltenen Reaktionsmischung wird anschließend, gegebenenfalls nach Abtrennung des Katalysators und Abfiltrieren von Feststoffen das Aryl-di- und/oder -polyurethan isoliert, beispielsweise durch Abdestillieren des überschüssigen O-Alkylcarbamidsäureesters und/oder Alkohols, durch partielles Abdestillieren des überschüssigen O-Alkylcarbamidsäureesters und/oder Alkohols und Auskristallisieren, durch Auskristallisieren, durch Ausfällen mit oder auch durch Umkristallisieren aus anderen Lösongsmitteln. Die Katalysatoren können gegebenenfalls abgetrennt werden, z.B durch Sedimentation, Abfiltrieren, Auswaschen oder Binden mit Ionenaustauschern.

Die Aryl-di- und/oder -polyurethane, können in an sich bekannter Weise in der Gasphase bei Temperaturen von 300° bis 600°C in Gegenwart von Katalysatoren beispielsweise analog den Angaben der DE-OS 19 44 719 (GB-PS 1 247 451), in flüssiger Phase in Gegenwart von Lösungsmitteln katalysatorfrei, beispielsweise analog den Angaben der DE-AS 24 21 503 (US 3 962 302) oder der DE-AS 25 30 001 (US 3 919 280) oder insbesondere in Gegenwart von Lösungsmitteln und Katalysatoren, beispielsweise analog den Angaben der DE-OS 26 35 490 bei Temperaturen von 175° bis 350°C thermisch in die entsprechenden Di- und/oder Polyisocyanate gespalten werden.

Es hat sich überraschend als vorteilhaft erwiesen, die thermische Spaltung durch Heterogenkatalyse mit Metallen aus der Reihe Zink, Aluminium, Titan, Eisen, Chrom, Kobalt und Nickel bzw. diese, gegebenenfalls in Kombination mit anderen Metallen, z.B. Vanadium und Wolfram, enthaltende Legierungen in oberflächenreicher Form, z.B. in Form von Metall-pulvern, -granulaten mit durchschnittlichen Durchmessern von 1 bis 10 mm, -spänen oder -wolle, zu beschleunigen. Die Metalle sind nicht nur gute Wärmeüberträger, sondern sie haben darüber hinaus einen guten, technisch vorteilhaft verwertbaren Katalyseeffekt und ermöglichen deutliche Senkungen der Reaktionstemperaturen und/oder -zeiten, so daß Nebenreaktionen, wie Polymerisationen eine geringere Rolle spielen.

Durch eine vorzügliche Aktivät zeichnen sich besonders Zink und Aluminium aus. Bei der Spaltung von Aryldiurethanen in der Gasphase erreicht man z.B. mit der Zink- und Aluminium-Heterogenkatalyse für die gleiche Spaltgeschwindigkeit eine Senkung der Spalttemperatur von 50° bis 100°C gegenüber bekannten Verfahren. Unter "Gasphase" im Sinne der Erfindung wird hierbei die vorher gennante Definition verstanden.

Die Katalysatoren können in veschiedenen Anordnungen angewandt werden, beispielsweise als Festbett, z.B. durch Beschicken von verschieden gestalteten Reaktoren, wie Rohr-, Tank-, oder Kesselreaktoren mit Metallgranulaten, -ringen, -spänen, oder -wolle, so daß das Reaktionsgemisch kontinuierlich durch das Katalysator-Festbett geleitet werden kann, oder auch in Suspension in Rührreaktoren.

Als Lösongsmittel können gegebenenfalls solche verwendet werden, die gegenüber den isocyanaten und übrigen Komponenten unter den Reaktionsbedingungen inert sind und sich im Siedepunkt von diesen unterscheiden, Lösungsmittel, deren Siedepunkte zwischen den Siedepunkten der Aryl-di-und/oder -polyisocyanate und des abgespaltenen Alkohols liegen, sind zweckmäßig für Spaltungen in flüssiger Phase, wenn das Di- und/oder Polyisocyanat als nicht destillierte Bodenfraktion isoliert wird; sie können aber auch bei Spaltungen in der Gasphase als Verdünnungsmittel Anwendung finden und dort gegebenenfalls die Isolierung des Isocyanats durch fraktionierende Kondensationen ohne Rekombination mit dem Alkohol erleichtern. Lösungsmittel, deren Siedepunkt höher liegt als die Siedepunkte der Spaltprodukte, werden bevorzugt für die Spaltung von Di- und/oder Polyurethanen in flüssiger Phase zu Di- und/oder Polyisocyanaten, die durch Destillation gegebenenfalls unter Mithilfe von Strippmitteln isoliert werden. Gegebenenfalls kann hierbei zu einer verbesserten Isolierung von Alkohol und Isocyanat zusätzlich ein zwischen Alkohol und Isocyanat siedendes Lösungsmittel eingesetzt werden. Besonders bewährt haben sich und vorzugsweise zur Anwendung kommen Dibenzylnaphthaline, die durch Benzylierung von Naphthalin mit Benzylchlorid zugänglich sind, für Spaltungen von Di- und/oder Polyurethanen in flüssiger Phase zu Aryl-di und/oder -polyisocyanaten, deren Siedepunkt tiefer liegt als der Siedepunkt des Dibenzylnaphthalins.

Vorteilhaft ist, wenn die Aryl-di- und/oder -polyurethane in dem Lösongsmittel löslich sind, obgleich dies nicht unbedingt notwendig ist. Das Lösungsmittel ist gleichzeitig Wärmemedium, das dazu dient, dem Reaktionssystem Wärme zuzuführen und die Reaktionstemperatur gleichmäßig zu gestalten.

Als Lösungsmittel seien beispielsweise genannt: aliphatische Kohlenwasserstoffe, wie die höheren Alkane Decan, Undecan, Dodecan, Tridecan, Tetradecan, Pentadecan, Hexadecan, Heptadecan, Dekalin, flüssiges Paraffin, Erdölfraktionen von Paraffinen, die überlicherweise als Schmieröle, Kühlöle oder Schneidöle verwendet werden; alicyclische Kohlenwasserstoffe, wie Erdölfraktionen der Naphthenreihen; gegebenenfalls substituierte aromatische Kohlenwasserstoffe, wie z.B. Naphthalin, 1- und 2-Methylnaphthalin, 1,2-, 1,4-, 1,6-, 2,7-, 2,6- und 2,3-Dimethylnaphthalin, 1-Äthylnaphthalin, Phenylnaphthalin, Benzylnaphthalin, Toluol, 1,2-, 1,3- und 1,4-Dimethylbenzol, 1,2,4- und 1,3,5-Trimethylbenzol, 1,2,3,5- und 1,2,4,5-Tetramethylbenzol, 1,3,5-Triäthylbenzol, Hexyl, Heptyl, Octyl, Nonyl, Decyl- und Dodecylbenzol, Hexamethylbenzol, Hexaäthylbenzol, Diphenyl, 4,4'-Dimethyldiphenyl, Dibenzyl, Diphenylmethan und 4,4'-Dimethyl-diphenylmethan, halogensubstituierte aromatische Kohlenwasserstoffe, wie z.B. Chlorbenzol, 1,2- und 1,4-Dichlorbenzol, 1,4-Dijodbenzol, 1,2,3- und 1,3,5-Trichlorbenzol, 1,2,3,4- 1,2,3,5- und 1,2,4,5-Tetrachlorbenzol, Pentachlorbenzolen 1- und 2-Fluornaphthalen, 1- und 2-Chlornaphthalin, 1- und 2-Jodnaphthalin und Diphenyl-dichlormethan; Nitrogruppen haltige aromatische Kohlenwasserstoffe, wie z.B. Nitrobenzol, 3-Nitrotoluol, 2-Nitro-m-xylol, 5-Nitro-m-xylol und 4-Nitroanisol, aliphatische und aromatische Ketone, wie z.B. Cyclohexanon, Cycloheptanon, Di-n-butylketon, Di-n-amylketon, α-Tetralon, Acetophenon, Propionphenon, Benzophenon, 3-Methylbenzophenon, Dodecanon-2 und Tridecanon-2, Sulfone und Carbonsäureester, wie z.B. Sulfolan, Diäthylsulfon, Phthalsäuredimethylester, Phthalsäurediäthylester, Benzoesäurepropylester und Laurinsäureäthylester und Äther, wie z.B. Diäthylenglykol-dimethyläther, Diäthylenglykol-diäthyläther, Diisoamyläther, Di-n-amyläther, Resorcindimethyläther, Resorcindiäthyläther, Phenyloctyläther, Phenylbenzläther, Dibenzyläther, Diphenyläther, α-Methylnaphthyläther und β-Äthylnaphthyläther.

Man kann die thermische Spaltung der Di- und/oder Polyurethane sowohl in der Gasphase als auch in flüssiger Phase diskontinuierlich oder kontinuierlich bei vermindertem, normalen oder erhöhtem Druck durchführen. Die Spaltung und Trennung der Produkte durch Abdestillieren das Alkohols und gegebenenfalls des Di- und/oder Polyisocyanats und/oder des Lösongsmittels können gleichzeitig oder

nacheinander erfolgen. In der Gasphase spaltet man in der Regel unter vermindertem Druck, dessen Maximum bei vorgegebener Temperatur gleich ist dem Siedepunkt des Reaktionsgemisches. In flüssiger Phase wählt man bei simultaner Spaltung und Trennung zweckmäßig eine Temperatur/Druck-Verhältnis, das dem Siedepunkt der tiefsiedenden Komponente der Bodenfraktion entspricht. In geschlossenem System, z.B. einem Rohrspaltreaktor, erfolgt die Spaltung zweckmäßigerweise ohne Erhöhung des Systemdrucks.

Mann kann die Ausgangsstoffe dampfförmig, geschmolzen oder in fester Form z.B. als Pulver, als Suspension oder als Lösung in einem inerten Lösungsmittel dem Reaktor zuführen, der wahlweise auf einer bestimmten Temperatur und einem bestimmten Druck gehalten wird. Man führt z.B. ein Aryldiurethan — dampfförmig, flüssig oder fest — entsprechend 0,1 bis 20, vorzugsweise 1 bis 10 Urethanäquivalente pro Liter und Stunde bei einer Temperatur von 300° bis 400°C, vorzugsweise von 330° bis 380°C und einem Druck von 1 mbar bis 1 bar, vorzugsweise von 1 bis 100 mbar in einen mit Zinkspänen beschickten Rohrreaktor und kondensiert das Aryl-diisocyanat, den Alkohol und gegebenenfalls nicht umgesetztes Aryldiurethan zweckmäßig unter Zufuhr eines inerten Lösungsmittels, dessen Siedepunkt zwischen den Siedepunkten des Alkohols und des isocyanates liegt, über eine Kolonne fraktionierend. Mann kann die Lösung eines Di- und/oder Polyurethans in einem inerten Lösungsmittel bei einer Temperatur von 175° bis 350°C, vorzugsweise 220° bis 320°C durch einen mit Zink- oder Aluminiumgranulaten beschickten Rohrreaktor und das Produkt anschließend zur Trenung in eine Kolonne oder durch eine Kaskade aus mehreren Spaltreaktoren und Trennkolonnen in abwechselnder Anordnung leiten. In einer anderen bevorzugten Ausführungsform führt man die Lösung kontinuierlich in einen Spaltreaktor bzw. eine Reaktorkaskade unter gleichzeiter Abtrennung des Alkohols, gegebenenfalls mit Hilfe eines Strippmittels oder Zwischensieders, und des Di- und/oder Polyisocyanates über eine oder mehrere Trennkolonnen unter Rückfluß des Lösungsmittels, das als Bodenfraktion abgezogen wird. In einer weiteren bevorzugten Verfahrensvariante kann man das Aryl-di- und/oder -polyurethan z.B. roh-MDU in einem geeigneten Lösungsmittel wie oben beschrieben in flüssiger Phase spalten, gleichzeitig den Alkohol unter Rückfluß des Lösungsmittels abdestillieren und anschließend aus dem Sumpf das Lösungsmittel durch schonende Destillation gegebenenfalls unter Strippen mit kurzer Verweilzeit unter Rückfluß des Arylk-di- und/oder -polyisocyanates abdestillieren und das Aryl-di- und/oder polyisocyanat als Bodenfraktion abführen. Hierbei hat es sich als vorteilhaft gezeigt, einen Anteil des Aryl-di- und/oder -polyisocyanates mit dem Lösungsmittel abzudestillieren und mit diesem zusammen im Kreislauf in den Spaltreaktor zurückzuführen.

Nach dem erfindungsgemäßen Verfahren können aromatische Di- und/oder Polyisocyanate mit hoher Reinheit in großen Ausbeten wirtschaftlich vorteilhaft hergestellt werden. Die Vorteile des erfindungsgemäßen Verfahrens werden besonders deutlich am Beispiel des roh-MDI, das erstmals ohne Verwendung von Phosgen Kohlenmonoxid une toxischen bzw. teueren Katalysatoren in der gewünschten Variationsbreite, insbesondere bezüglich der Isomerenverteilung, dadurch gewonnen werden kann, daß man die Kondensation von Anilin mit Formaldehyd in bekannter Weise zu der gewünschten Isomerenverteilung steuert und das so erhaltene Gemisch aus Diaminodiphenylmethanen und Polyphenyl-polymethylen-polyaminen (roh-MDA) nach dem erfingungsgemäßen Verfahren in roh-MDI überführt.

Die erfindungsgemäß erhaltenen aromatischen Di- und/oder -polyisocyanate werden vorzugsweise zur Herstellung von Polyurethan-Kunststoffen verwendet.

Die in den Beispielen genannten Teile beziehen sich auf das Gewicht.

## Beispiel 1

61 Teile 2,4-Diaminotoluol werden mit 250 Teilen Carbamidsäureäthylester, 6 Teilen Eisen-(II)-acetat und 140 Teilen Äthanol 14 Stunden lang auf 180 bis 190°C erhitzt, wobei über ein Druckregelventil im Reaktionsgefäß ein Druck von 8 bis 10 bar eingestellt wird. Das während der Reaktion gebildete Ammoniak wird unter Verwendung von 30 l Stickstoff je l Reaktionsgemisch und Stunde als Schleppmittel kontinuierlich abdestilliert. Nach beendeter Reaktion analysiert man die Reaktionslösung gaschromatographisch nach der Methode des "Internen Standard" sowie durch Hochdruck-Flüssigchromatographie nach der Methode des "Externen Standard". Es zeigt sich, daß 94% des eingesetzten 2,4-Diaminotoluol umgesetzt werden, wobei 113 Teile 2,4-Di-(äthoxicarbonylamino)-toluol (90.4% der Theorie, bezogen auf umgesetztes 2,4-Diaminotoluol) entstanden sind. Weiterhin befindet sich in der Reaktionslösung noch ein Gemisch von 2-Amino-4-(äthoxicarbonylamino)-toluol und 4-Amino-2-(äthoxicarbonylamino)-toluol.

Man destilliert aus der Reaktionslösong überschüssiges Äthanol und überschüssigen Carbamidsaüreäthylester ab, löst den Rückstand in 1 000 Teilen Methylenchlorid und wäscht ihn mehrmals mit Wasser. Dann entfernt man das Methylenchlorid, gibt 200 Teile Äthanol hinzu und stellt in eine Eis-Kochsalz-Mischung. Nach einiger Zeit kristallisieren 85 Teile (68% der Theorie, bezogen auf umgesetztes 2,4-Diaminotoluol) 2,4-Di-(äthoxicarbonylamino)-toluol vom Schmp. 107 bis 109°C aus. Das so erhaltene Diurethan wird über eine Pulverdosiereinrichtung mit einer Rate von ca. 200 Litern pro Liter Reaktionsraum und Stunde in einem auf 360°C erhitzten, mit Zinkspänen gefüllten Rohrreaktor aus Quarzglas kontinuierlich eingeführt. Im Spaltreaktor wird ein Druck von 15 bis 25 mbar aufrechtgehalten. Die austretenden Spaltgase werden fraktioniert kondensiert, wobei in einer mit Wasser gekühlten Vorlage 49.5 Teile Toluylendiisocyanat (TDI) (89.0% der Theorie, bezogen auf eingesetztes 2,4-Di-(äthoxicarbonyl-amino)-toluol) erhalten werden. In der Vorlage kann gaschromatographisch noch ein Gemisch aus 4-(äthoxicarbonylamino)-toluylen-2-isocyanat und 2-(äthoxicarbonylamino)-toluylen-4-isocyanat nachgewiesen werden.

Beispiel 2

61 Teile 2,4-Diaminotoluol werden mit 450 Teilen Carbamidsäuremethylester, 5 Teilen Zinkacetat, 60 Teilen Harnstoff und 192 Teilen Methanol 16 Stunden lang auf 180 bis 190°C erhitzt, wobei über ein Druckregelventil im Reaktionsgefäß ein Druck von 11 bis 13 bar eingestellt wird. Des während der Reaktion gebildete Ammoniak wird unter Verwendung von 25 l Stickstoff je l Reaktionsgemisch und Stunde als Schleppmittel kontinuierlich abdestilliert. Nach beendeter Reaktion analysiert man die Reaktionslösung gaschromatographisch nach der Methode des "Internen Standard" sowie durch Hochdruck-Flüssigchromatographie nach der Methode des "Externen Standard". Es zeigt sich, daß 98% des eingesetzten 2,4-Diaminotoluols umgesetzt werden, wobei 97 Teile 2,4-Di-(methoxicarbonylamino)-toluol (83.2% der Theorie, bezogen auf umgesetztes 2,4-Diaminotoluol) entstanden sind. In der Reaktionslösung befindet sich noch ein Gemisch aus 2-Amino-4-(methoxicarbonylamino)-toluol und 4-Amino-2-(methoxicarbonylamino)-toluol. Man destilliert aus der Reaktionslösung überschüssiges Methanol und überschüssigen Carbamidsäuremethylester ab. löst den Rückstand unter Erwärmen in 500 Teilen Methanol, filtriert von ungelöstem Rückstand ab und läßt in einer Eis-Kochsalz-Mischung erkalten. Nach einiger Zeit kristillisieren 86 Teile 2,4-Di-(methoxicarbonylamino)-toluol vom Schmp. 158 bis 162°C aus.

Das so erhaltene Diurethan wird über eine Pulverdosiereinrichtung mit einer Rate von ca. 250 Litern pro liter Reaktionsraum und Stunde in einen aus 350°C erhitzten, mit Zinkspänen gefüllten Rohrreaktor aus Quarzglas kontinuierlich eingeführt. Im Spaltreaktor wird ein Druck von 10 bis 15 mbar aufrechtgehalten. Die austretenden Spaltgase werden fraktioniert kondensiert, wobei in einer mit Wasser gekühlten Vorlage 60 Teile Toluylendiisocyanat (TDI) (95.4% der Theorie, bezogen auf eingesetztes 2,4-Di-(methoxicarbonylamino)-toluol) erhalten werden.

Beispiel 3

40 Teile 2,4-Diaminotoluol werden mit 240 Teilen Caramidsäurehexylester, 1,5 Teilen Kobaltacetat und 170 Teilen Hexanol 15 Stunden lang auf 155 bis 175°C erhitzt, wobei das entstehende Ammoniak kontinuierlich abdestilliert wird. Nach beendeter Reaktion untersucht man die Reaktionslösung durch Hochdruck-Flüssigchromatographie nach der Methode der "Externen Standard". Es zeight sich, daß das 2,4-Diaminotoluol vollständig umgesetzt ist, wobei 119 Teile 2,4-Di-(hexoxicarbonylamino)-toluol (96% der Theorie, bezogen auf umgesetztes 2,4-Diaminotoluol) entstanden sind.

Der beim Erkalten angefallene Katalysator wird abgetrennt. Nach Waschen des Filtrats mit Wasser destilliert man überschüssiges Hexanol und überschüssiges Carbamidsäurehexylester unter vermindertem Druck ab. Man erhält 134 Teile eines Destillationsrückstandes, der in 300 Teilen Dibenzylnaphthalin gelöst wird. Diese Lösung gibt man mit einer Zulaufrate von 350 Litern pro Liter Reaktionsraum und Stunde in einen auf 320°C erhitzten, mit Aluminiumgranulat gefüllten Rohrreaktor aus Quarzglas. Das bei der Reaktion gebildete Hexanol wird unter Verwendung von 35 l Stickstoff je l Reaktionsgemisch und Stunde gasförming abgetrennt und in mehreren hintereinandergeschalteten wassergekühlten Vorlagen kondensiert. Man erhält 358 Teile Reaktionsaustrag, aus dem durch Vakuum-destillation bei 76 bis 82°C und 0,2 mbar 53 Teile Toluylendiisocyanat (92.9% der Theorie, bezogen auf eingesetztes 2,4-Diaminotoluol) erhalten werden.

Beispiel 4

100 Teile eines handelsüblichen Roh-MDA-Gemisches, das zu 56% aus Diamino-diphenylmethan und zu 44% Polyphenyl-polymethylen-polyaminen besteht, wird mit 30,3 Teilen Harnstoff, 300 Teilen Carbamidsäurehexylester, 1,5 Teilen Kobaltacetat und 260 Teilen Hexanol 25 Stunden lang auf 155 bis 175°C erhitzt, wobei das entstehende Ammoniak kontinuierlich abdestilliert wird. Nach beendeter Reaktion untersucht man die Reaktionslösung durch hochdruck-Flüssigchromatographie, wobie sich zeigt, daß ein Gemisch aus Di-(hexoxicarbonylamino)-diphenylmethanen und Poly-(hexoxicarbonylamino)-polyphenyl-polymethanen entstanden ist, welches identisch ist mit einem aus "Roh-MDI" und Hexanol hergestellten Vergleichsprodukt.

Man läßt den Katalysator absetzen, wäscht nach Filtration mit Wasser und destilliert überschüssiges Hexanol und überschüssigen Carbamidsäurehexylester im Vakuum ab. Es werden 233 Teile Destillationsrückstand erhalten, die in 750 Teilen Decylbenzol gelöst werden. Man gibt diese Lösung mit einer Zulaufrate von 300 Litern pro l Reaktionsraum und Stunde in einen auf 300°C erhitzten, mit Zinkspänen gefüllten Rohrreaktor aus Quarzglas. Das bei der Spaltung gebildete Hexanol wird gasförmig abgetrennt und in mehreren wassergekühlten Vorlagen kondensiert. Aus dem Reaktionsaustrag destilliert man bei 1 bis 2 mbar das Spaltlösungsmittel kontinuierlich so ab, daß der Sumpf praktisch lösungsmittelfrei ist. Das Destillat enthält hierbei ungefähr 0,4% Diphenylmethandiisocyanat.

Man erhält 113 Teile Destillationsrückstand, der aus einem Gemisch von 51% Diphenylmethandiisocyanat und 49% Polyphenyl-polymethylen-polyisocyanat ("Roh-MDI") besteht.

Beispiel 5

50 Teile 4,4'-Diaminodiphenylmethan werden 360 Teilen Carbamidsäurehexylester, 1 Teil Kobaltacetat und 260 Teilen Hexanol 35 Studen lang auf 155 bis 175°C erhitzt. Nach dem Erkalten filtriert man vom

abgesetzten Katalysator ab und analysiert die Reaktionsmischung durch Hochdruck-Flüssigchromatographie nach der Methode des "Externen Standard". Es zeigt sich, daß das 4,4'-Diamino-diphenylmethan vollständig umgesetzt ist. Nach Waschen des Reaktionsaustrags mit Wasser destilliert man überschüssiges Hexanol und überschüssigen Carbamidsäurehexylester ab. Man erhält 121 Teile Destillationsrückstand, der in 120 Teilen 1,2,4,5-Tetramethylbenzol gelöst wird. Diese Lösung gibt man mit einer Zulaufrate von 100 Litern pro 1 Reaktionsraum und Stunde in einen auf 350°C erhitzten, mit Aluminiumspänen gefüllten Rohrreaktor aus Quarzglas, in dem ein Druck von 5 bis 10 mbar aufrecht gehalten wird. Das bei der Spaltung gebildete Hexanol wird gasförmig abgetrennt und mit verdampftem Tetramethylbenzol in einer mit Wasser gekühlten Vorlage kondesiert. Als Reaktionsaustrag erhält man 93 Teile einer Lösong von 51 Teilen 4,4'-Diphenylmethan-diisocyanat (80.8% der Theorie, bezogen auf eingesetztes 4,4'-Diamino-diphenylmethan) in Tetramethylbenzol.

Beispiel 6

15:8 Teile 1,5-Diaminonaphthalin werden mit 89 Teilen Carbamidsäureäthylester, 12 Teilen Harnstoff, 0,15 Teilen Kobaltacetat und 46 Teilen Äthanol 12 Stunden lang auf 180° erhitzt, wobei über ein Druckregelventil im Reaktionsgefäß ein Druck von 8 bis 10 bar eingestellt wird. Das während der Reaktion gebildete Ammoniak wird unter Verwendung von 20 l Stickstoff je l Reaktionsgemisch und Stunde als Schleppmittel kontinuierlich abdestilliert. Nach beendeter Reaktion werden überschüssiges Äthanol, überschüssiger Carbamidsäureäthylester und nicht umgesetztes 1,5-Diaminonaphthalin teilweise im Vakuum abdestilliert. Nach Zugabe von 450 Teilen Äthanol stellt man in eine Eis-Kochsalz-Mischung, wobei nach einiger Zeit 25 Teile 1,5-Di-(äthoxicarbonylamino)-naphthalin (82.8% der Theorie, bezogen auf eingesetztes 1,5-Diaminonaphthalin) vom Schmp. 217 bis 223°C auskristallisieren. Das so erhaltene 1,5-D-(äthoxicarbonylamino)-naphthalin wird über eine Pulverdosiereinrichtung mit einer Rate von 450 Litern pro l Reaktionsraum und Stunde in einen auf 350°C erhitzten, mit Zinkspänen gefüllten Rohrreaktor eingeführt. Im Spaltreaktor wird ein Druck von 1 bis 3 mbar aufrechtgehalten. Die austretenden Spaltgase werden fraktioniert kondensiert, wobei in einer ersten mit Wasser gekühlten Vorlage 14 Teile 1,5-Naphthylendiisocyanat (NDI) (80.5% der Theorie, bezogen auf eingesetztes, 1,4-Di-(äthoxicarbonylamino)-naphthalin) vom Schmelzpunkt 129 bis 132°C erhalten werden.

**Patentansprüche**

1. Verfahren zur Herstellung von aromatische Di- und/oder Polyisocyanaten, dadurch gekennzeichnet, daß man

A. primäre aromatische Di- und/oder Polyamine mit O-Alkylcarbamidsäureestern in Gegenwart oder Abwesenheit von Katalysatoren und gegebenenfalls Harnstoff und Alkohol bei erhöhten Temperaturen zu Aryl-di- und/oder -polyurethanen umsetzt und das dabei gebildete Ammoniak gegebenenfalls abtrennt und

B. die erhaltenen Aryl-di- und/oder -polyurethane durch themische Spaltung in Gegenwart von Katalysatoren bei Temperaturen von 175° bis 600°C in aromatische Di- und/oder Polyisocyanate überführt.

2. Verfahren nach Anspruch 1, dadurch gekennzeichnet, daß man die Aryl-di- und/oder -polyurethane (A) in Gegenwart mindestens einer Verbindung, die ein oder mehrere Kationen von Metallen aus den Gruppen IA, IB, IIA, IIB, IIIA, IIIB, IVA, IVB, VA, VB, VIB, VIIB und VIIIB des Periodensystems besitzt, als Katalysator herstellt.

3. Verfahren nach Ansprüchen 1 bis 2, dadurch gekennzeichnet, daß man zur Herstellung der Aryl-di- und/oder -polyurethane (A) die Ausgangskomponenten in solchen Mengen umsetzt, daß das Verhältnis von $NH_2$-Gruppen der aromatischen Di- und/oder Polyamine zu O-Alkylcarbamidsäureester zu gegebenenfalls Alkohol 1:0,5 bis 20:0 bis 100 beträgt.

4. Verfahren nach Ansprüchen 1 bis 3, dadurch gekennzeichnet, daß man zur Herstellung der Aryl-di- und/oder -polyurethane (A) neben O-Alkylcarbamidsäureester zusätzlich Harnstoff und Alkohol mitverwendet, wobei das Verhältnis von Harnstoff zu Alkohol gleich oder kleiner als 1 ist.

5. Verfahren nach Ansprüchen 1 bis 4, dadurch gekennzeichnet, daß man zur Herstellung der Aryl-di- und/oder -polyurethane (A) neben O-Alkylcarbamidsäureester maximal 1,5-Äquivalente Harnstoff, bezogen auf $NH_2$-Gruppen der Di- und/oder Polyamine, einsetzt.

6. Verfahren nach Ansprüchen 1 bis 5, dadurch gekennzeichnet, daß man zur Herstellung der Aryl-di- und/oder -polyurethane als aromatische Di- und/oder Polyamine 2,4- und 2,6-Diaminotoluol und die entsprechenden Isomerengemische, 1,5-Diaminonaphthalin, 3,3'-Ditoluylen-4,4'-diamin, 2,2'-, 2,4'- und 4,4'-Diaminodiphenylmethan und die entsprechenden isomerengemische sowie Mischungen aus Diamino-diphenylmethan-Isomeren und Polyphenyl-polymethylen-polyaminen verwendet.

7. Verfahren nach Anspruch 1, dadurch gekennzeichnet, daß man als O-Alkylcarbamidsäureester solche aus Carbamidsäure und aliphatischen und cycloaliphatischen Monoalkoholen mit 1 bis 10 Kohlenstoffatomen im Alkoholrest verwendet.

8. Verfahren nach Anspruch 1, dadurch gekennzeichnet, daß man die Aryl-di- und/oder -polyurethane (B) in flüssiger Phase in einem Lösungsmittel bei Temperaturen von 175° bis 350°C in Gegenwart von in heterogener Phase vorliegenden Metallen aus der Reihe Zink, Aluminium, Titan, Eisen, Chrom, Kobalt und Nickel als katalysatoren thermisch spaltet.

9. Verfahren nach Anspruch 8, dadurch gekennzeichnet, daß man zur thermischen Spaltung in

flüssiger Phase ein Lösungsmittel verwendet, dessen Siedepunkt zwischen dem des gebildeten Di- und/oder Polyisocyanats und dem des abgespaltenen Alkohols liegt.

10. Verfahren nach Anspruch 8, dadurch gekennzeichnet, daß man als Katalysator Aluminium oder Zink verwendet, die in der heterogenen Phase in einer oberflächenreichen Form vorliegen.

11. Verfahren nach Anspruch 1, dadurch gekennzeichnet, daß man die Aryl-di- und/oder -polyurethane (B) in der Gasphase bei Temperaturen von 300° bis 600°C in Gegenwart von Zink oder Aluminium, thermisch spaltet.

12. Verfahren nach Anspruch 11, dadurch gekennzeichnet, daß das Zink oder Aluminium in einer oberflächenreichen Form vorliegt.

13. Verfahren nach Anspruchen 1 und 8, dadurch gekennzeichnet, daß man die Aryl-di- und/oder -polyurethane in flüssiger Phase thermisch spaltet, einen Teil des erhaltenen Aryl-di- und/oder -polyisocyanates mit dem Lösungsmittel abdestilliert und mit diesem zusammen im Kreislauf in den Spaltreaktor zurückführt und den restlichen Teil der Aryl-di- und/oder -polyisocyanate als Bodenfraktion abführt.

**Revendications**

1. Procédé de préparation de di- et/ou poliisocyanates aromatiques, caractérisé en ce que

A. on fait réagir des di- et/ou polyamines aromatiques primaires sur des carbamates de O-alkyle, en présence ou en l'absence de catalyseurs et éventuellement d'urée et d'un alcool, à des températures élevées, de façon à obtenir des aryl-di- et/ou polyuréthanes et on sépare éventuellement l'ammoniac formé au cours de cette réaction et

B. on transforme les aryl-di- et/ou-polyuréthanes obtenus par scission thermique, en présence de catalyseurs, à des températures variant de 175 à 600°C, en di- et/ou polyisocyanates aromatiques.

2. Procédé suivant la revendication 1, caractérisé en ce que l'on prépare les aryl-di- et/ou-polyuréthanes (A) en présence d'au moins un composé qui possède un ou plusieurs cations de métaux choisis parmi les groupes IA, IB, IIA, IIB, IIIA, IIIB, IVA, IVB, VA, VB, VIB, VIIB et VIIIB du système périodique des éléments, à titre de catalyseur.

3. Procédé suivant l'une quelconque des revendications 1 et 2, caractérisé en ce qu'en vue de la préparation des aryl-di- et/ou-polyuréthanes (A) on fait réagir les constituants de départ en proportions telles que le rapport des groupes $NH_2$ des di- et/ou polyamines aux carbamates de O-alkyle et éventuellement à l'alcool varie de 1:0,5 à 20:0 à 100.

4. Procédé suivant l'une quelconque des revendications 1 à 3, caractérisé en ce qu'en vue de la préparation des aryl-di- et/ou-polyuréthanes (A), on utilise complémentairement de l'urée et un alcool au carbamate de O-alkyle, le rapport de l'urée à alcool étant égal ou inférieur à 1.

5. Procédé suivant l'une quelconque des revendications 1 à 4, caractérisé en ce qu'en vue de la préparation des aryl-di- et/ou-polyuréthanes (A), on utilise, complémentairement au carbamate de O-alkyle, au maximum 1,5-équivalent d'urée, par rapport aux groupes $NH_2$ des di- et/ou polyamines.

6. Procédé suivant l'une quelconque des revendications 1 à 5, caractérisé en ce qu'en vue de la préparation des aryl-di- et/ou -polyuréthanes, on utilise, en tant que di- et/ou polyamines aromatiques, le 2,4- et le 2,6-diamino-toluène et les mélanges d'isomères correspondants le 1,5-diaminonaphtalène la 3,3'-ditoluylène-4,4'-diamine, le 2,2'-2,4'- et 4,4'-diaminodiphénylméthanes et les mélanges d'isomères correspondants, comme aussi des mélanges d'isomères du diaminodiphénylméthane et de polyphénylpolyméthylène-polyamines.

7. Procédé suivant la revendication 1, caractérisé en ce qu'à titre de carbamates de O-alkyle, on utilise ceux provenant de l'acide carbamique et de monoalcools aliphatiques et cycloaliphatiques dont le reste alcool comporte de 1 à 10 atomes de carbone.

8. Procédé suivant la revendication 1, caractérisé en ce que l'on soumet les aryl-di- et/ou -polyuréthanes (B) à une scission thermique, en phase liquide, dans un solvant, à des températures de 175 à 350°C, en présence de métaux de la série du zinc, de l'aluminium, du titane, du fer, du chrome, du cobalt et du nickel, qui se présentent en phase hétérogène, à titre de catalyseurs.

9. Procédé suivant la revendication 8, caractérisé en ce qu'en vue de la scission thermique en phase liquide, on utilise un solvant dont le point d'ébullition fluctue entre celui du di- et/ou polyisocyanate engendré et celui de l'alcool séparé.

10. Procédé suivant la revendication 8, caractérisé en ce qu'à titre de catalyseur, on utilise de l'aluminium ou du zinc qui se présentent en phase hétérogène sous une forme dotée d'une grande surface.

11. Procédé suivant la revendication 1, caractérisé en ce que l'on soumet les aryl-di- et/ou -polyuréthanes (B) à une scission thermique en phase gazeuse, à des températures de 300 à 600°C, en présence de zinc ou d'aluminium.

12. Procédé suivant la revendication 11, caractérisé en ce que le zinc ou l'aluminium se présentent sous une forme dotée d'une grande surface.

13. Procédé suivant l'une quelconque des revendications 1 et 8, caractérisé en ce que l'on soumet les aryl-di- et/ou polyuréthanes à une scission thermique en phase liquide, on chasse une partie des aryl-di- et/ou -polyisocyanates obtenus avec le solvant par distillation et on les renvoie ensemble avec ce dernier dans le réacteur de scission et on évacue la partie résiduelle des aryl-di- et/ou -polyisocyanates sous forme de fraction de queue.

**Claims**

1. A process for the preparation of an aromatic di- and/or polyisocyanate, wherein

A. a primary aromatic di- and/or polyamine is reacted with an O-alkyl carbamate in the presence or absence of a catalyst and in the presence or absence of urea and alcohol at elevated temperature to give an aryl di- and/or polyurethane, and the ammonia formed in the reaction may or may not be separated off, and

B. the resulting aryl di- and/or polyurethane is converted by thermal cleavage, in the presence of a catalyst, at a temperature of from 175 to 600°C into an aromatic di- and/or polyisocyanate.

2. A process as claimed in claim 1, wherein the aryl di- and/or polyurethane is prepared in the presence of at least one compound containing one or more cations of metals selected from groups IA, IB, IIA,. IIB, IIIA, IIIB, IVA, IVB, VA, VB, VIB, VIIB and VIIIB of the periodic table, as catalyst.

3. A process as claimed in claims 1 and 2, wherein, to prepare the aryl di- and/or polyurethanes (A), the starting components are reacted in such quantities that the ratio of $NH_2$ groups of the aromatic di- and/or polyamines to O-alkyl carbamate to any alcohol present is 1:0.5 to 20:0 to 100.

4. A process as claimed in claims 1 to 3, wherein, to prepare the aryl di- and/or polyurethanes (A), urea and alcohol are co-used in addition to O-alkyl carbamate, the ratio of urea to alcohol being equal to or less than 1.

5. A process as claimed in claims 1 to 4, wherein, to prepare the aryl di- and/or polyurethanes (A), a maximum of 1.5 equivalents of urea, based on the $NH_2$ groups of the di- and/or polyamines, is used in addition to O-alkyl carbamate.

6. A process as claimed in claims 1 to 5, wherein, to prepare the aryl di- and/or polyurethanes (A), 2,4- and 2,6-diaminotoluene and the corresponding isomer mixtures, 1,5-diaminonaphthalene, 3,3'-ditoluene-4,4'-diamine, 2,2'-, 2,4'- and 4,4'-diaminodiphenylmethane and the corresponding isomer mixtures, as well as mixtures of diaminodiphenylmethane isomers and polyphenyl polymethylene polyamines are used as aromatic di- and/or polyamines.

7. A process as claimed in claim 1, wherein the O-alkyl-carbamates used are produced from carbamic acid and aliphatic and cycloaliphatic monoalcohols having 1 to 10 carbon atoms in the alcohol radical.

8. A process as claimed in claim 1, wherein the aryl di- and/or polyurethanes (B) are thermally cleaved in the liquid phase in a solvent at a temperature of from 175 to 350°C in the presence of metals selected from the group consisting of zinc, aluminum, titanium, iron, chromium, cobalt and nickel which are present in the heterogeneous phase, as catalysts.

9. A process as claimed in claim 8, wherein a solvent is used for the thermal cleavage in the liquid phase, whose boiling point is located between that of the resulting di- and/or polyisocyanate and that of the separated alcohol.

10. A process as claimed in claim 8, wherein there is used as catalyst aluminum or zinc which in the heterogeneous phase has a large surface area.

11. A process as claimed in claim 1, wherein the aryl di- and/or polyurethane (B) is thermally cleaved in the gas phase at a temperature of from 300 to 600°C in the presence of zinc or aluminum.

12. A process as claimed in claim 11, wherein the zinc or aluminum has a large surface area.

13. A process as claimed in claims 1 and 8, wherein the aryl di- and/or polyurethane is thermally cleaved in the liquid phase, part of the resulting aryl di- and/or polyisocyanate is distilled off with the solvent and recycled with the solvent to the cleaving reactor, and the remaining part of the aryl di- and/or polyisocyanate is discharged as bottom fraction.